# EUROPEAN PATENT SPECIFICATION

(11) **EP 1 105 515 B1**
(45) Date of publication and mention of the grant of the patent: **28.06.2006**
(21) Application number: 99941028.5
(22) Date of filing: 10.08.1999
(51) Int. Cl.: C12P 19/24, C12N 9/10, C12N 15/00, C07H 21/04

(54) **ALPHA 2,8/2,9 POLYSIALYLTRANSFERASE**
ALPHA-2,8/2,9-POLYSIALYLTRANSFERASE
ALPHA 2,8/2,9 POLYSIALYLTRANSFERASE

(30) Priority: 10.08.1998 US 96003 P
(43) Date of publication of application: 13.06.2001
(73) Proprietor: THE SCRIPPS RESEARCH INSTITUTE, La Jolla, CA 92037 (US)
(72) Inventor: WONG, Chi-Huey, Rancho Santa Fe, CA 92067 (US); SHEN, Gwo-Jenn, Carlsbad, CA 92009 (US); DATTA, Arun, San Diego, CA 92130 (US)
(74) Representative: Hedley, Nicholas James Matthew
(86) International application number: PCT/US1999/018154
(87) International publication number: WO 2000/009736

(56) References cited:
- VIMR E R ET AL: "HOMOLOGY AMONG ESCHERICHIA COLI K1 AND K9 POLYSIALYLTRANSFERASE" , JOURNAL OF BACTERIOLOGY, WASHINGTON, DC, US, VOL. 174, NR. 15, PAGE(S) 5127-5131 XP002923349 ISSN: 0021-9193 * abstract * * page 5127, column 1, paragraph 1 * * page 5127, column 2, paragraph 2 * * page 5128, column 1, paragraph 2 * * figure 2 *
- STEENBERGEN S M ET AL: "FUNCTIONAL ANALYSIS OF THE SIALYTRANSFERASE COMPLEXES IN ESCHERICHIA COLI K1 AND K92" , JOURNAL OF BACTERIOLOGY, WASHINGTON, DC, US, VOL. 174, NR. 4, PAGE(S) 1099-1108 XP002923348 ISSN: 0021-9193 * abstract * * page 1101, column 2, paragraphs 2,3 * * page 1106, column 2, paragraph 2 *
- CHAO C-F ET AL: "ON THE BIOSYNTHESIS OF ALTERNATING ALPHA-2,9/2,8 HETEROPOLYMER OF SIALIC ACID CATALYZED BY THE SIALYLTRANSFERASE OF ESCHERICHIA COLI BOS-12" THE JOURNAL OF BIOLOGICAL CHEMISTRY, vol. 274, no. 26, 25 June 1999 (1999-06-25), pages 18206-18212, XP001080196
- TROY F A: "POLYSIALYLATION: FROM BACTERIA TO BRAINS" , GLYCOBIOLOGY, IRL PRESS,, GB, VOL. 2, NR. 1, PAGE(S) 5-23 XP002923347 ISSN: 0959-6658 * page 5, column 2, paragraph 1 * * table 1 *
- TROY: 'Polysialylation: from bacteria to brains.' MINI REVIEW, DEPARTMENT OF BIOLOGICAL CHEM. 1992, pages 15 - 23, XP002923347
- STEENBERGEN ET AL.: 'Functional analysis of the sialyltransferase complexes in Escherichia coli K1 and K92' J. BACTERIOL., vol. 174, no. 4, 1992, pages 1099 - 1108, XP002923348
- VIMR ET AL.: 'Homology among Escherichia coli K1 and K92 polysialyltransferases' J. BACTERIOL., vol. 174, no. 15, 1992, pages 5127 - 5131, XP002923349

## Description

### Technical Field:

The invention relates to α2,8/2,9 polysialyltransferase and to methods for producing and using recombinant α2,8/2,9 polysialyltransferase.

### Background:

The 9-carbon 5-amino-3,5-dideoxy-D-glycero-D-galacto-nonulosonic acids or sialic acids, comprise a family of neuraminic acid derivatives which are widly spread in nature, ranging from bacterial to human origin. This sugar moiety linked to carbohydrate chains of glycolipids and glycoproteins plays a key role in many important biological events mediated by carbohydrate-protein interactions (Schauer, R. et al. (1995) *Biology of the Sialic acids,* pp 7-67, Plenum Press, New York., Rosenberg, A. ed). Sialic acids are also found in linear homopolymers (polySia) of *N*-acetylneuraminic acid (Neu5Ac) and *N*-glycolylneuraminic acid (Neu5Gc) joined by α2,8-, α2,9- or α2,8/α2,9-ketosidic linkages (Troy, F.A. (1992) *Glycobiology* **2,** 5-23). The degree of polymerization can extend beyond 200 sialic acid residues (Rohr, T.E. et al. (1980) *J*. *Biol. Chem.,* **255,** 2332-2342.), thereby constituting a structurally unique group of carbohydrate polymers which covalently modify cell-surface glycoconjugates (Troy, F.A. (1990) *Trends Glycosci. Glycotechnol.* **2,** 430-449). Polysialylation of the mammalian neural cell adhesion molecules (NCAM), for example, affects the cell-cell adhesive interactions during embryogenesis, including neurite fasciculation and neuromuscular interactions (Edelman, G. M.. (1985) *Cold Spring Harbor Symp. Quant. Biol.* **50:** 877-889; and Cunningham, B. A. et al. (1987) *Science* **236:** 799-806). While the neuroinvasive bacteria *Escherichia coli* K1 contain a homopolymer of (-8-NeuAcα2-) (Troy, F. A. et al. (1979) *J. Biol. Chem.* **254,** 7377-7387), the *Neisseria meningitidis* Group C (Liu, T.-Y. et al. (1971) *J. Biol. Chem.* **246:** 4703-4712; and Vann, W. F. et al. (1978) *J. Bacteriol.* **133,** 1300-1306) displays a homopolymer of (-9-NeuAcα2-) linkages. On the other hand, the polySia capsular antigens of neuroinvasive *E*. *coli* K92 are composed of poly (-8NeuAcα2,9NeuAcα2-) residues (Egan, W. et al. (1977) *Biochemistry,* **16,** 3687-3692). Alternating linkages of α2,8NeuAc- and α2,9NeuAc- could not be found in mammalian systems, and therefore could be utilized to elicit immunological response. In fact, the heteropolymer from the K92 strain of *E.coli* is being imployed as a carbohydrate vaccine in the clinical trial of patients suffering from acute meningitidis (Devi, S. J. et al. (1991) *Proc Natl Acad Sci* U S A, **88,** 7175-9).

The sialyltransferases that catalyze the addition of sialic acid to form such diverse carbohydrate recognition molecules fall into at least two families. While 13 clones have been obtained from mammalian systems (Tsuji, S. et al. (1996) *Glycobiology* **6,** v-vii), only 4 have been cloned from bacterial systems to date (Vimr, E. R. et al. (1992) *J. Bacteriol* **174,** 5127-5131; Gilbert, M.. et al. (1997) *Eur. J. Biochem.* **249:** 187-194; and Yamamoto T. et al. (1998) *J. Biochem* (Tokyo) **123,** 94-100). Recent cloning of both mammalian and bacterial sialyltransferases showed that these two gene families evolved differently. The polysialyltransferase (polyST) encoded by *neu*S in *E.coli* uses CMP-sialic acid as a donor and catalyzes a sequential addition of sialic acid to the nonreducing end of an acceptor (Kundig, F. D. et al. (1971) *J. Biol Chem.* **246:** 2543-2550; and Steenbergen, S.M. et al. (1990) *Molecular Microbiology* **4**: 603-611). In a detailed study using the pathogenic strain of *E.coli* K1, the gene coding for CMP-Neu5Ac: poly-α2,8 sialosyl sialyltransferase, termed *neuS,* has been cloned. This gene was found to be clustered in the *kps* region which is implicated in the initiation, polymerization and possibly translocation of polySia chains across the inner membrane (Weisgerber, C. et al. (1991) *Glycobiology* **1,** 357-365; Weisgerber, C. et al. (1990) *J. Biol. Chem.* **265,** 1578-1587; and Steenbergen, S.M. et al. (1991) *Glycoconjugate J.* **8,** 145). A similar gene has been identified by hybridization to the K92 strain *of E.coli,* the capsule of which contains poly (-8NeuAcα2,9NeuAcα2-) (ibid, Egan, W. et al. 1977) . The complete nucleotide sequence of this K92 neuS gene showed that this gene product differs by 70 amino acids from the K1 neuS gene product (ibid, Vimr, E. R. 1992). Though an *in vitro* study indicated that the neuS from *E. Coli* K1 coding for a 47 kd protein has polysialyltransferase activity, no such activity has yet been clearly demonstrated for the neuS product from E. coli K92.

### Summary:

Disclosed herein is the cloning of the neuS gene from the K92 strain of *E.coli* by PCR using the primers described previously (ibid, Vimr, E. R. et al. 1992), expressed the gene in *E*. *coli,* and shown that the enzyme from this gene catalyzes the synthesis of polysialic acids with both α2, 8 and α 2, 9-linkages *in vitro* and *in vivo.*

One aspect of the invention is directed to DNA plasmid containing the neuS gene from *escherichia coli* K92 and encoding α2, 8/2, 9 polysialyltransferase from *escherichia coli* K92, the α2, 8/2, 9 polysialyltransferase having a hexameric histidine tag fused to its N-terminal end.

Another aspect of the invention is directed to a transformed cell having a gene encoding α2, 8/2, 9 polysialyltransferase having a hexameric histidine tag fused to its N-terminal end from *escherichia coli* K92, the gene being as set out in the preceding paragraph.

Another aspect of the invention is directed to a process for obtaining α2, 8/2, 9 polysialyltransferase having a hexameric histidine tag fused to its N-terminal end. In the first step of the process, a transformed cell having a gene for α2, 8/2, 9 polysialyltransferase having a hexameric histidine tag fused to its N-terminal end expresses such gene. The expressed α2, 8/2, 9 polysialyltransferase having a hexameric histidine tag fused to its N-terminal end is then released from the cell. The α2, 8/2, 9 polysialyltransferase may be from *escherichia coli* K92.

Another aspect of the invention is directed to recombinant α2, 8/2, 9 polysialyltransferase that is unbound to a cell membrane and that has a hexameric histidine tag fused to its N-terminal end. In a preferred embodiment, the α2, 8/2, 9 polysialyltransferase is from *escherichia coli* K92.

Another aspect of the invention is directed to a process for converting a substrate of α2, 8/2, 9 polysialyltransferase into a product. In this process, the substrate is contacted with α2, 8/2, 9 polysialyltransferase that has a hexameric histidine tag fused to its N-terminal end and that is unbound to a cell membrane under conditions for promoting enzymic catalysis of a conversion of the substrate into the product. In a preferred mode, the α2, 8/2, 9 polysialyltransferase is from *escherichia coli* K92.

### Brief Description of the Figures:

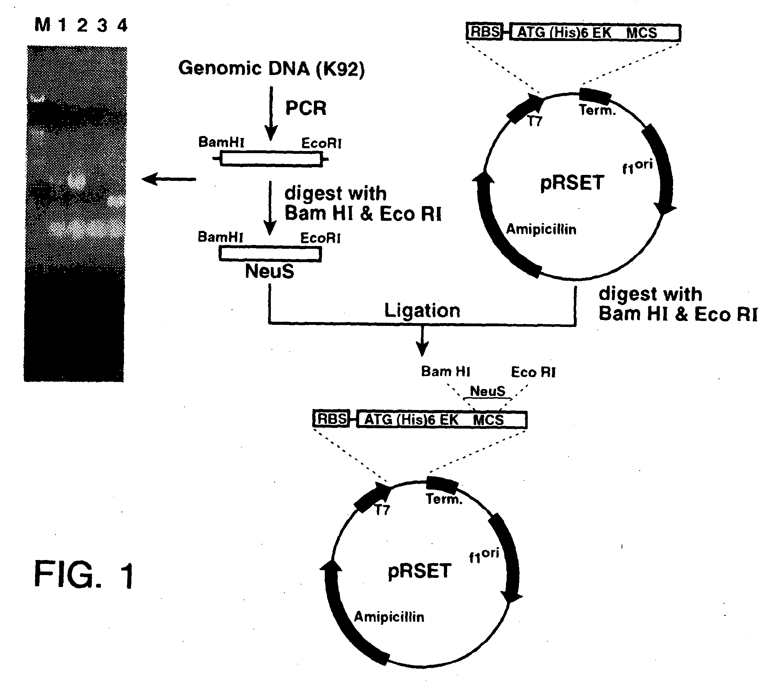

Figure 1 illustrates the construction of the expression vector for the K92 neuS expression as a fusion protein. The PCR fragment 91. 2 kb obtained for the full-length form of the K92 neuS gene using its genomic DNA as template, was subcloned in pRSET vector (Novagen, Madison, WI) by using two unique restriction sites Barn HI and Eco RI. This resulted in the expression of the protein with a hexameric histidine tag fused at its N-terminal end. The vector provides the start codon followed by the sequence for the hexameric histidine. The full length form of the protein remains mostly as membrane bound form. However, about 30-40% of the protein is "released" in the 40K Sup upon treatment with a detergent. This released protein retains full catalytic activity and was used in this study.

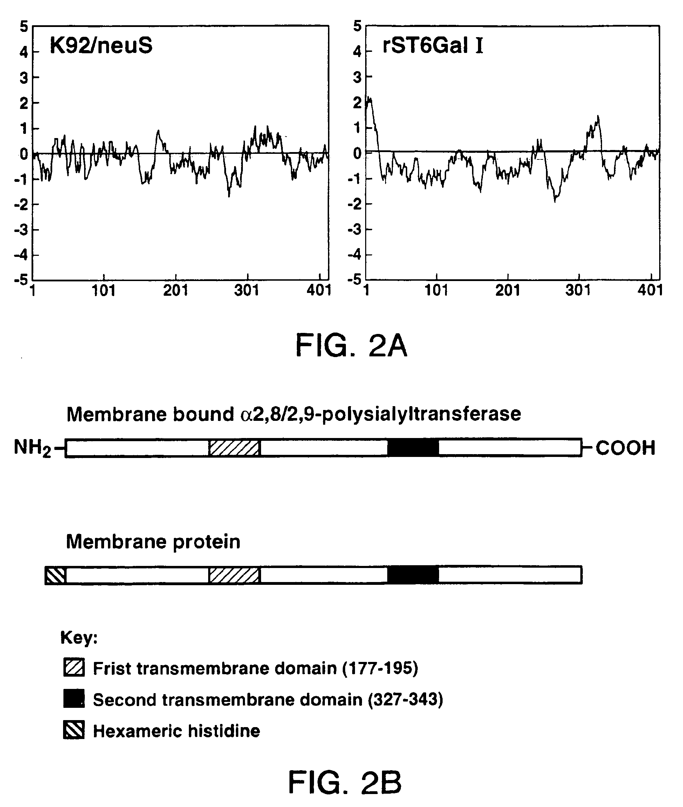

Figure 2 illustrates a hydrophilicity plot and the predicted secondary structure for the α2, 8/2, 9 polysialyltransferase. The hydrophilicity plot of the full length form of the K92 neuS gene product suggests the presence of two predicted membrane retention signals in the middle of the polypeptide. The occurrence of this protein (both native and recombinant full-length form) as membrane bound form also supports this model. It is disclosed that approximately 30-40% of the protein is "released" in the 40K Sup using suitable detergent. This released protein retains full catalytic activity and was used in this study. The hydrophilicity plot was compared with that of mammalian ST6Gal I (Weinstein et al 1987) as a representative. This enzyme is predicted to contain only one membrane spanning domain.

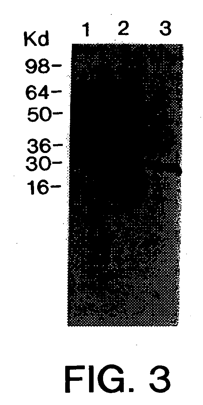

Figure 3 illustrates a Western blot analysis of the K92 neuS expressed protein. *E.coli* cells of BL21(DE3) transformed with K92 *neu*S gene in the expression vector pRSET were induced with IPTG (1 mM final concentration) and harvested after 4 hrs. Cells transformed with pRSET vector only were also grown and used as negative control. As a positive control, BL21(DE3) previously transformed with the histidine tagged thioesterase gene (40) subcloned in pET20B(+) were grown, induced, and harvested similarly. After harvesting, the cells were lysed and the supernatants were collected after centrifugation at 15,000 rpm (15K Sup). The proteins were run using a 12% SDS-PAGE as mentioned in the 'Materials and Methods'. The samples were: 15K Sup from the negative control (lane 1), 15K Sup from the cells transformed with K92 *neu*S gene (lane 2), and 15K Sup from the positive control (lane 3). The blot was developed using anti-(His)₅ antibody (Qiagen) and metal enhanced DAB substrate kit (Pierce). The neuS expressed α2,8/2,9-polysialyltransferase migrated in the gel at about 47.5 kd. In lane 3, the positive control for the Western blot showed a band at ~ 23 kd for the thioesterase. It may be noted that the expression level for the *neu*S gene was comparatively lower than the thioesterase gene.

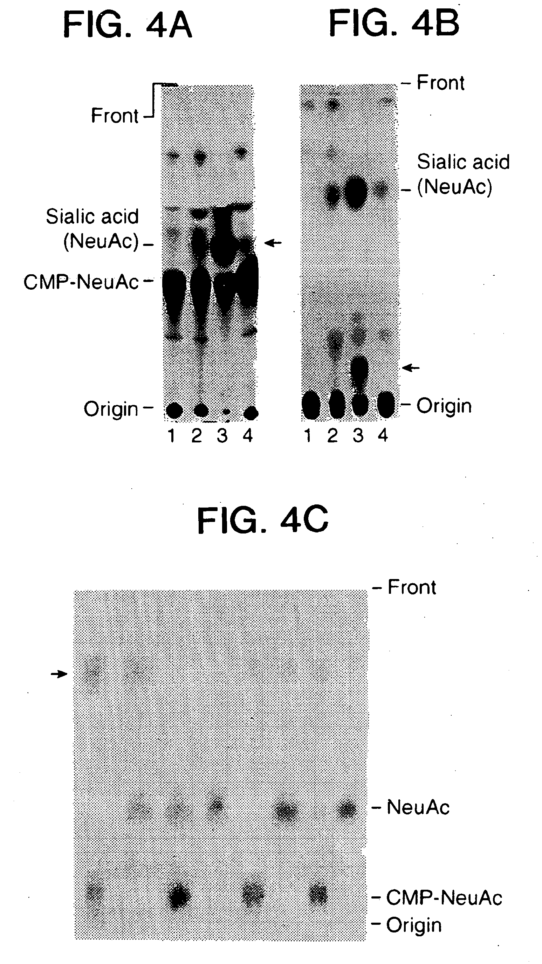

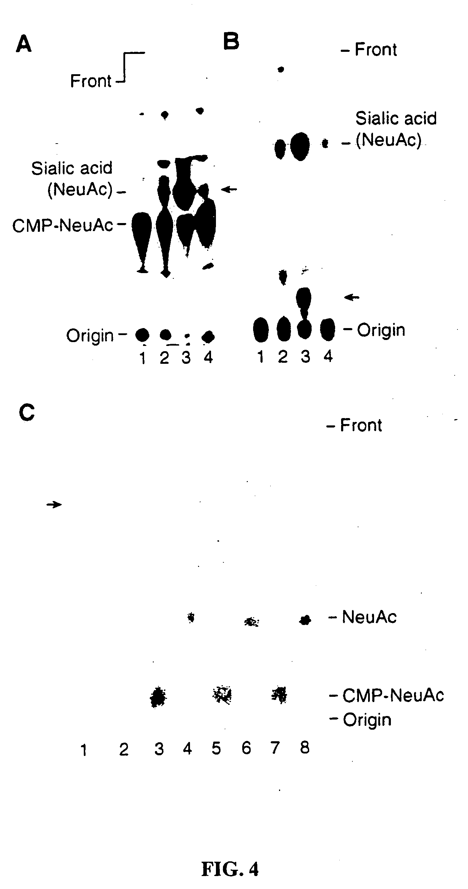

Figure 4 illustrates an analysis of the enzyme reaction products by thin-layer chromatography (TLC). The 40K Sup was used as a source of the enzyme in the reaction mixture containing 1 mM acceptor and CMP[¹⁴C]NeuAc (9 nmol; 1. (Ci) as donor in a reaction buffer containing 25 mM Tris-HCl (pH 8.0), 5 mM MgCl₂, 5 mM DTT, and 0.5% Triton CF-54. As an example, analysis was shown with the reaction mixture containing sialic acid and GM₃ as acceptors and CMP[¹⁴C]NeuAc as donor substrate. As a negative control, the reaction mixture contained sialic acid as acceptor without the addition of any enzyme. For A and B, silica gel 60 TLC plates were used for the samples: reaction mixture without the enzyme (lane 1), GM₃ as acceptor (lane 2), sialic acid as acceptor (lane 3), and reaction mixture without exogenous sialic acid (lane 4). The solvents used were: (A) *n*-propanol:1 M NH₄OH: water:: 7 : 1: 2; (B) chloroform : methanol: 0.02%CaCl₂:: 45: 55: 10. The reaction mixtures were also analyzed by DEAE-cellulose TLC. After the reaction was complete, a part of the sample was acidified with 10% acetic acid and incubated for 30 min at room temperature. For C, DEAE-cellulose TLC plate was used. The samples were: reaction mixture with sialic acid as acceptor (lane 1) and after acidification (lane 2); reaction mixture without enzyme (lane 3) and after acidification (lane 4); using GM₃ as acceptor (lane 5) and after acidification (lane 6); reaction mixture without exogenous acceptor (lane 7) and after acidification (lane 8). The solvents in C used were: *n*-propanol: 14.5 M NH₄OH: water:: 6: 1: 3. The plates were dried after the run and exposed to a Kodak' XAR-5 film. The arrow mark indicates the position of the potential cell free reaction product(s). The position of CMP-NeuAc and free sialic acid were also shown. We found that both the enzyme and its reaction products are unstable and degrade over time.

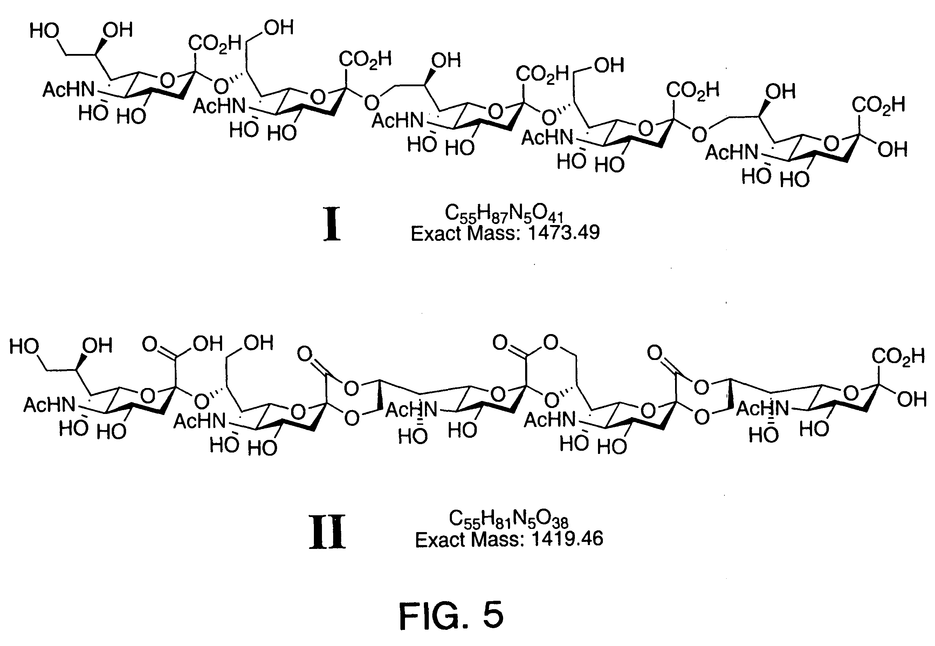

Figure 5 illustrates the structure of the cell-free reaction product. The product obtained by the cell-free reaction using K92 *neu*S protein, indicated the structure of a pentameric sialic acid with alternating α2,9/2,8-linkages (I). The proposed major lactone form (II) obtained in the enzymatic reaction is also shown.

### Detailed Description:

The abbreviations used herein are: NeuAc, neuraminic acid; PCR, polymerase chain reaction; CMP-NeuAc, CMP-neuraminic acid or CMP-sialic acid; nt., nucleotide(s); TLC, thin layer chromatography.

### Cloning of K92 neuS gene:

The genomic DNA isolated from *Escherichia coli* strain K92 (ATCC 35860) using a DNA extraction kit (Qiagen Co., Valencia, CA) was used for the amplification of *neu*S gene by PCR. This was performed in a 100 (1 reaction mixture containing 100 ng of genomic DNA as template, 300 nmoles of primers, 200 mM of dNTPs, 50 mM KCl, 10 mM Tris-HCl (pH 8.3), 2 mM MgCl₂, 0.01% gelatin, 0.1% Triton X-100 and a mixture of both *Tag* DNA polymerase and *pfu* DNA polymerase (1 unit of each). Two primers, the forward primer with internal *Bam* H1 (underlined) site [5'ATATTGGATCCATATTTGATGCTAGTT TA 3'; nt. 3 - 20] and a reverse primer with *Eco* R1 site, (5'GGCGCGAATTCTTACTC CCCCAAGAAAA 3'; nt. 1230 - 1214) designed based on the previously published sequence (ibid, Vimr, E.R. 1992) were used for PCR using the following conditions: 94 °C, 1 min; 50 °C, 1 min, and 72 °C, 2 min for 35 cycles. Agarose gel analysis of the product showed the presence of one major band with the expected size of about 1.2 kb. The fragment obtained was purified by agarose gel electrophoresis, digested with *Bam* HI and *Eco* RI and subcloned into a similarly digested pRSET vector (Invitrogen Co., San Diego, CA) following standard molecular biological techniques (Sambrook, J. et al. (1989) *Molecular cloning: A Laboratory Manual,* Cold Spring Harbor Laboratory, Cold Spring Harbor, NY.). The ligation mixture was than used for transformation of competent cells of the BL21(DE3) strain of *E.coli* (Novagen, Madison, WI). The clone containing the expression vector was confirmed by sequencing of both strands using an automated DNA sequencer (ABI 377) at the TSRI Core facility.

### Expression of the K92 neuS gene.

Since the DH5α strain of *E.coli* lacking the *kps* gene cluster does not have sialyltransferase activity (ibid, Steenbergen, S.M. 1990), it was used intially to express the gene. This strain has a pilli forming gene in its chromosome and therefore does not require any pressure for the expression of its sex pilli. M13 phage containing the T7 RNA polymerase gene (Invitrogen) was used to infect DH5αF' transformed with pRSET vector containing the cloned *neu*S gene. The transformed cells were grown in LB medium (with 1 mM MgCl₂) containing 100 (g carbicillin/ml at 37 °C to O.D.₆₀₀ ~0.3 and then 1 mM IPTG was added. After one hour of incubation, the M13/T7 phage was added to infect the cells as described by the supplier (Invitrogen), and harvested after 5 hours of incubation. An enzyme activity assay and SDS-PAGE of the cell lysate (15 K supernatant) showed expression of the *neuS* gene in DHSαF'. A band migrating as ~ 47.5 kd in the 12% SDS-PAGE appeared within 1 hour of incubation after infection with T7 RNA polymerase gene containing M13 in the presence of IPTG (as judged by Western blot). The relative intensity of the 47.5 kd band increased with time and reached maximum after 4 hrs. Enzymatic assay of the cell free extract using sialic acid as acceptor also indicated the expression of the K92 polysialyltransferase. However, the DHSaF' cells transformed with pRSET vector also yielded a band comigrating with the 47. 5 kd band in SDS-PAGE. Attempt to separate these bands using Ni2+-NTA agaxose proved to be difficult. We therefore decided to express this protein in the HMS 174 (DE3) Lys or BL21(DE3) strain of E. coli (Invitrogen).

The transformed BL21 (DE3) cells containing the neuS gene from the K92 strain of E. coli were grown exponentially in LB medium (with 1 mM MgCl2) containing 100 (g carbicillin/ml at 37 C until A600-0.3 was reached. Expression of the neuS gene was achieved at 37 C by induction with IPTG as described in the expression kit (Invitrogen, Carlsbad, CA). The cells were harvested after 5 hours of incubation.

A DNA plasmid designated as BL21/DE3 (8, 9 PST) containing the neuS gene from K92, obtained above, was originated from escherichia coli K92 strain by PCR to clone the a2, 8/2, 9 polysialyltransferase. The DNA was subcone in pRSET vestor (Invitrogen) and the resultant plasmid DNA (8, 9 PST) was transformed into escherichia coli GL21(DE3).

### Western blot:

This analysis was done by following a standard technique (Harlow, E, et al. (1988) Antibodies, Cold Spring Harbor Laboratory, Cold Spring Harbor, NY. ). The protein samples were boiled for 8-10 mins in 1 x Laemmli gel sample buffer either in the absence or presence of 10% -mercaptoethanol. Electrophoresis was performed using 10% SDS-polyacrylamide gel in Tris/glycine-SDS buffer. After the proteins were transferred to a nitrocellulose membrane, the blot was blocked with a blocking buffer (blotto in TBS and 0. 1% Tween-20) and developed by adsorption with the mouse anti- (His) g monoclonal antibody (Qiagen, Valencia, CA) diluted (1 : 500) with the blocking buffer. The blot was incubated with horseradish peroxidase-conjugated sheep anti-mouse secondary antibody (1 : 1000 ; Amersham Corp. ). The protein bands were visualized either by chemiluminescence as suggested by the supplier of the reagents (Amersham Corporation) or by staining with the immunoPure Metal Enhanced DAB Substrate Kit (Pierce, Rockford, IL).

### Sialyltransferase assay:

The assay was carried out essentially as described by Vimr et al (Steenbergen, S. M. et al. (1992) J. Bacteriol. 174 : 1099-1108) with the following modifications : after harvesting, the cells were washed three times with 25 mM Tris HCI (pH 8. 0) and then resuspended in 25 mM Tris HCl (pH 8. 0) containing 5 mM MgCl2, 5 mM dithiothreitol (DTT), and 1 (1/mL protease inhibitor cocktail (Sigma). Cells were lysed three times by using a French Press (AMINCO ; 1, 500 psi). The cell debris was removed by centrifugation twice at 15, 000 rpm and the supernatant was collected (termed the 15 K Sup). This supernatant was then centrifuged again at 100,000x g (~ 40 K rpm) using a Beckman ultracentrifuge (Model L5-50). Both the supernatant (termed the 40 K Sup) and the pellet were saved. The pellet was resuspended in a buffer containing 25 mM Tris-HCI, 5 mM MgCl₂, 5 mM DTT, and 0.1% Triton CF-54. The supernatants and the pellet were stored on ice, and used for assay within 2 h.

Without the addition of acceptor, the endogenous enzyme activity was determined. The exogenous activity was then detected by addition of sialic acid as acceptor and CMP[¹⁴C]NeuAc as donor in a reaction buffer containing 25 mM Tris-HCl (pH 8.0), 5 mM MgCl₂, 5 mM DTT, and 0.5% Triton CF-54. Other substrates were also used, including colominic acid. Both the endogenous and exogenous enzyme activity assays measure the transfer of ¹⁴C-labeled NeuAc to the acceptor. Unreacted radiolabeled substrates were seperated by DEAE-cellulose TLC (n-propanol/14M NH₄OH/H₂O:: 6 : 1: 3) or by Sephadex G-25 column chromatography. Radioactivity remaining at the origin and/or at the top of the TLC plate or the product obtained at the void volume of Sephadex G-25 column, was quantitated by liquid scintillation spectrometry. For analysis using Sephadex G-25 column chromatography, a column (1 cm x 3.5 cm) of Sephadex G-25 (Pharmacia) in water was prepared. The radiolabeled cell-free reaction mixture (60 (1) was loaded and washed with 1.5 ml of water, which was collected and counted. In some experiments, the activity was determined by separating the unused CMP[¹⁴C]NeuAc by Dowex 1X8 (phosphate form) column chromatography (Williams, M. A. et al. (1995) *Glycoconj. J.* **12**: 755 - 761). The initial rates were estimated graphically, and activity was expressed as pmoles of sialic acid transferred per hour.

### Thin-layer chromatography and fluorography:

After the cell-free reaction was complete, the proteins were removed by an Amicon filter (MWCO 10 K), and the supernatant containing oligomeric reaction products was separated by Silica gel 60 (aluminum backed) and/or DEAE-cellulose (plastic backed) thin-layer chromatography (TLC) using the following solvent systems: (a) *n*-propanol, 14 M NH₄OH, H₂O :: 6: 1: 3; (b) chloroform : methanol: 0.02% CaCl₂:: 30 : 55: 15. Control chromatograms were calibrated with mono- to hexasialosyl oligomers (Calbiochem, San Diego, CA) which were detected by orcinol spray, as described by Corfield and Schauer *(Sialic Acids: Chemistry, Metabolism, and Function.* (Schauer, R., ed) *Cell Biology Monograph,* Vol **10**, pp. 77-94, Springer-Verlag, New York, 1982). This method resolved monomeric through octameric sialic acids. Oligomers composed of nine or more sialosyl residues were retained at the origin. Labeled chromatograms were sprayed with En³Hance (New England Nuclear) and fluorographed against an intensifier screen. After fluorography, sialosyl oligomers were detected on Silica gel 60 TLC plates by orcinol spray for alignment of radioactive signals with colorimetrically detected bands. For analysis using DEAE-cellulose TLC, the position of the cell-free ¹⁴C-labeled reaction product(s) was marked after alignment of radioactive signals, cut and counted.

### Isolation and purification of polysialylated oligosaccharides:

Strains of *Escherichia coli* transformed with vector only or with the K92 *neu*S gene, were used for the extraction of polysaccharide following the method of Kasper et al (Kasper, D. L. et al. (1973) *J. Immunol.* **110**, 262-268) with the following modifications. For K92 polysaccharide, the K92 strain of *E.coli* was grown in 2 L of Davis' supplemental minimal medium (ATCC media formulation # 54) at 37 °C overnight. The K1 strain of *E.coli* transformed with K92 *neu*S gene was grown in 2 L of Davis' supplemental minimal medium containing 2% glycerol instead of glucose. The cells were grown to O.D.₆₀₀ ~ 0.3 and 1 mM IPTG (final concentration) was added. After incubation at 37 °C for 1 hr., M13/T7 phages (Invitrogen; 10 pfu/cell) were added and incubated at 37 °C. After overnight growth the organisms were removed by centrifugation (48,000 x g for 30 min at 4 °C), and the supernatant was collected and filtered through a 0.45 ( Millipore filter. Hexadecyltrimethyl ammonium bromide (Cetavlon; 0.3%; Sigma) was added to the supernatant with stirring and the precipitate formed was collected by centrifugation. The precipitate was solubilized in 0.9 M CaCl₂, and reprecipitated with three volumes of chilled (-20 °C) absolute ethanol. The ethanol precipitation from 0.9 M CaCl₂ was repeated three times. The final product was dialyzed against water and lyophilized. The final purification was done using a column (2 cm x 25 cm) of Biogel P2 (Pharmacia) eluted with water following usual procedure, and verified by polyacrylamide gel electrophoresis following the method of Troy and McCloskey (ibid 1979). After hydrolysis by sulfuric acid (0.1 N H₂SO₄, 80 °C), quantitation of sialic acid was determined by the thiobarbituric acid procedure as described previously (Liu, J. et al. (1992) *J. Am. Chem. Soc.* **114**: 3901-3910).

### Protein Assay:

This was done using bicinconic acid (BCA) reagent following the instructions provided by the supplier (Pierce Chemical Co.).

### Mass spectrometry-analysis:

The electrospray ionization (ES-MS) mass spectrometry experiments were performed on an API III Perkin Elmer SCIEX triple quadrupole mass spectrometer. Electrospray samples were typically introduced to the mass analyzer at a rate of 4.0 ml/minute. The positive and negative ions, generated by charged droplet evaporation, enter the analyzer through an interface plate and a 100 mm orifice, while the declustering potential is maintained between 50 - 200 V to control the collisional energy of the ions entering the mass analyzer. The emitter voltage is typically maintained at 5000 V. The m/z was determined for the major peaks.

### NMR analysis:

¹³C Nuclear magnetic resonance spectra were recorded at ca. 23 °C on a Bruker AMX-400 or DRX-600 spectrometer operating in the pulsed Fourier transform mode using broadband proton noise decoupling. The polysaccharides were run as deuterium oxide solutions at pH 7.0. The solvent, D₂O, served as an internal lock signal. DMSO was often used as internal reference. The¹³C spectrum of polysaccharides from K1 and K92 determined in this study were found to be identical to that presented by Egan et al (ibid, Egan, W. 1977). The K1 polysaccharide NHCO (amide carbonyl) resonance from the present and Egan's studies were taken to have the same chemical shift and all remaining signals referenced accordingly.

### Results:

### Cloning of the neuS gene from K92:

Previously, the *neuS* gene from the K1 strain of *E.coli* was cloned and found to have the α2,8-polysialyltransferase activity. Using the K1 *neuS* gene as a probe, Vimr et al cloned a 1230 bp gene (ibid, Vimr, E.R. 1992) from the K92 strain of *E.coli* that had 87.3% homology to the K1 gene. The deduced protein sequence from this gene indicated the same number of amino acids (total 409 aa) with 83% homology to that of K1 at the protein level. Previous structural studies on the polysaccharide antigen of this *E. coli* K92 strain (Bos-12) suggested the presence of polysialic acid with alternating (-2,8 and (-2,9 linkages (ibid, Egan, W. 1977). Therefore, the gene product from the K92 strain is assumed to confer polysialyltransferase activity (14, 23). However, expression of the *neuS* gene from K92 was not clearly demonstrated, nor was it shown to form a polymer of sialic acid *in vitro* with alternating α2,8/2,9-linkages.

To characterize the K92 *neuS* gene product, we have cloned this gene from the *E. coli* K 92 strain using a PCR based approach. The primers were designed based on the previously published sequence (ibid, Vimr, E.R. 1992), and used to amplify the full-length clone of the *neuS* gene from the genomic DNA of the K92 strain. The 1.23 kb fragment obtained by PCR was subcloned in pRSET (Invitrogen) for expression of the *neuS* gene product as a fusion protein with the hexameric histidine tag fused at its *N*-terminal end (Fig. 1). The clone was verified by double-strand sequencing of the entire fragment including the restriction sites.

A hydrophilicity plot (Hopp, T. P. et al. (1981) *Proc. Natl. Acad. Sci.* U.S.A. 78, 3824-3828; and Kyte, J. et al. (1982) *J. Mol. Biol.* 157, 105-132) revealed two potential membrane-spanning regions in this protein of 409 amino acid residues. The first one is located 177 residues from the N-terminus. It consists of 19 hydrophobic amino acids bordered by three upstream lysine residues. Interestingly, this first transmembrane domain is bordered on either end by a proline residue. Proline is known to confer conformational constraints of many biologically important proteins, as evidenced by site-directed mutagenesis studies (ibid, Datta, A.K. 1998; for a review, see Vanhoop, G. et al. (1995) *FASEB J.* **9**, 736-744). A similar KKKP motif, found to be present in the ST6Gal I (for nomenclature, see Ref. 12) polypeptide, is part of a proposed signal anchor sequence (Weinstein, J. et al. (1987) *J. Biol. Chem.,* **262**, 17735-17743). The second transmembrane domain is located 327 residues from the *N*-terminus. It consists of 17 hydrophobic amino acid residues bordered by two upstream lysine residues and a proline residue at its C-terminus. Notably, lysine and serine are predominantly present in this domain. The presence of these two transmembrane domains sugests that this protein is membrane-bound as further supported by the Western blot (see below). This feature appears to be unique among the cloned bacterial sialyltransferases, the hydrophilicity plot of which suggests the presence of only one potential membrane-spanning region.

### Expression of the K92 neuS gene:

For expression, we used pRSET (Invitrogen), a prokaryotic expression vector designed to obtain a hexameric histidine tag fused at the *N*-terminal sequence of the desired protein. Due to the lack of any antibody available against the K92 *neuS* gene product, the polysialyltransferase was expressed as a fusion protein, and detected by the Western blot using anti-(His)₅ antibody (Qiagen Inc., Valencia, CA).

*SDS-Polyacrylamide gel electrophoresis and Western blot:* The cell free crude extract (15 K Sup and 40 K Sup) and the cell pellet, suspended in 25 mM Tris-HCl (pH 8.0) containing 5 mM MgCl₂ and 0.1% Triton CF-54, obtained by ultracentrifugation were analyzed by SDS-PAGE. Coumassie blue staining showed the presence of various bands including a major band at about 47.5 kd, the expected size for the *neuS* gene product. This band was absent in the control (cells transformed with pRSET vector only). Replica gel was used to transfer the proteins to a nitrocellulose membrane and detected by using mouse monoclonal anti-(His)₅ antibody (Qiagen, Valencia, CA). The blot was finally developed by using Enhanced DAB substrates following the protocol of the supplier (Pierce) or by chemiluminescence technique as described earlier (Datta, A. K. et al. (1998) *J. Biol. Chem.* **273**, 9608-9614). The Western blot clearly showed the presence of a single band at about 47.5 kd (Fig. 3). The α2,8/2,9-polysialyltransferase was also found to be present in the 40K supernatant. Our initial attempt to purify the protein using Ni²⁺NTA agarose column failed. Though the fusion protein apparently binds to the Ni²⁺NTA agarose, non-specific binding with other cellular proteins was also observed. Therefore, the 40K Sup was used in this study for enzyme assay and analysis of its product(s).

### Sialyltransferase assay:

The cell free crude extract (15 K Sup), 40 K supernatant (40 K Sup), and the pellet were used for the assay of various acceptor substrates and CMP [¹⁴C]NeuAc as donor substrate. The activity could be traced in all these fractions. After the reaction was over, the products were analysed by TLC or by Sephadex G-25 column chromatography. Fig. 4 shows a representative analysis. Using cold sialic acid as acceptor and CMP[¹⁴C]NeuAc as donor, a major spot was detected on TLC using various solvent systems. Interestingly, this new spot behaved as non-polar material on DEAE-cellulose TLC (Fig. 4C). It was found that sialic acid served as a good acceptor, whereas colominic acid did not (Table I). 9-*O*-acetyl sialic acid was not well accepted, indicating that the polymerization probably starts at the C9 position of the sialic acid acceptor. Notably, pentameric and hexameric polysialosides with α2,8-linkages were not accepted, whereas the corresponding dimer and trimer (Calbiochem) served as poor acceptors. Among the glycolipids tested, GD₃ and GQ_{1b} also served as good acceptors. However, other sialyl gangliosides tested were poor substrates (Table I).

### Structural determination of the cell -free reaction product :

For structural determination, the cell-free reactions were carried out, using the 40K supernatant as the enzyme source, with sialic acid and unlabeled CMP-NeuAc in a molar ratio of 1:10 to obtain the reaction product(s) in a sufficient quantity. Since it was known that the byproduct CMP inhibits the forward reaction, alkaline phosphatase was added to remove CMP in order to increase the yield. As mentioned above, the product moved as a non-polar material on DEAE-cellulose TLC, a characteristic utilized for purification of the cell-free reaction product(s). After the reaction was complete, the reaction mixture was passed through a column of Dowex 1X8 (phosphate form) to remove unreacted CMP-NeuAc and sialic acid. The negative ion mass spectral analysis (ES-MS.) showed a major peak at m/z 1419 for (M-3H₂O-H and at m/z 1401 for (M-4 H₂O-H). This spectrum was similar to that of the commercially obtained α2,8-polysialic acid (pentamer; Calbiochem). While the authentic sample showed a fragmentation pattern accounting for the tetramer- (*m*/*z* at 1182 for M- 3H₂O-H), trimer- (*m*/*z* at 890 for M-3H₂O-H), dimer- *(m*/*z* at 599 for M-3H₂O-H), and monomer-(m/z at 308 for M-3H₂O-H) fragments, the fragmentation pattern for the reaction product was slightly different, probably due to the presence of alternating α2,8 & α2,9-linkages in the cell-free reaction product(s). The degree of polymerization appears to be limited by the concentration of CMP-NeuAc. The production of a pentamer as the major product in the cell-free reaction is probably due to the limitation of CMP-NeuAc concentration along with the limited degree of polymerization. Increasing the amount of CMP-NeuAc in the cell-free reaction indeed increases the chain length of the sialyl polymer as judged by mass-spectrometry. While the MALDI-MS (Core Facility, The Scripps Research Institute) of the commercially available pentamer (Calbiochem) showed a peak for *mlz* at 1473, a peak at *m*/*z* 1474 was obtained with 10 mM CMP-NeuAc and at *m*/*z* 3830 with 20 - 30 mM CMP-NeuAc.

The *E.coli* strain K1 which forms the homopolymer of (-2,8-linked sialic acids in its capsule was transformed with the *neuS* gene from K92. This was done to investigate whether the K92 *neuS* gene product competes with the endogenous α2,8-polysialyltransferase for the same endogenous acceptor *in vivo.* The capsular polysaccharide from the transformed K1 was isolated and purified by Biogel P2 column chromatography as mentioned in 'Materials and Methods'. ¹³C-NMR of this polysaccharide in D₂O showed a spectrum different from that of the K1 and K92 derived polysialic acids. The major peaks for the NCOCH3 and NCOCH3 were similar to those of both K1 and K92. However, the signal for C-2 at δ103.44 was comparatively weaker. This may be due to the complex nature of the polysaccharide from transformed cells. Moreover, the ¹³C-NMR spectra obtained for the transformed K1 was similar to that of the polymer obtained from the K92 strain of *E.coli.,* which also showed two closely adjacent signals at δ 23.36 and 23.51. It was shown earlier that this particular strain of *E.coli* contains heteropolysialyl oligosaccharide with alternating α2,8/2,9-linkages (ibid, Egan W. 1977). This complexity was also evident in the ¹H-NMR spectrum which showed two peaks at δ1.99 and δ1.96. While a detailed structural study is in progress, the preliminary data indicates that the polysaccharide isolated from the transformed K1 has both α2,8- and α2,9-linkages present in random sequences. This suggests that not only is the K92 *neuS* gene functional in the K1 strain of *E.coli,* but also that its expression disrupted normal polysialyl chain elongation. This result is probably due to the competition for the same endogenous acceptor *in vivo.* This also suggests that the endogenous acceptor is neither sialic acid nor colominic acid, but is common for both the α2,8- and α2,8/2,9-polysialyltransferases.

Interestingly, the cell-free enzyme reaction product(s) appears to be prone to lactone formation. The radiolabeled product ran as a non-polar sialoside on DEAE-cellulose TLC and also passed through a column of Dowex 1X 8 (phosphate form). Lactone formation appears to depend on sample preparation conditions, and seems to be preferable at lower pH. This lactonization was also observed at pH 8.0, presumably because the pH of the reaction mixture decreased during the reaction. The electrospray mass analysis also suggested lactone formation of in the cell-free reaction product(s). The peak for *m*/*z* at 1401 is consistent with the loss of four H₂O to form 4 lactones and the major peak for *m*/*z* at 1419 is consistent with the formation of 3 lactones from the pentameric product. Additional supporting evidence for the presence of lactones is the ¹³C-peaks at -97ppm which are also observed in the authentic pentamer lactone prepared from the partial hydrolysis of the K92 polysaccharide. These peaks shift to ~103.5ppm when lactone hydrolysis under basic conditions is performed. Based on the recent observation of regioselective lactonization of (-2,8-linked oligomeric sialic acid (Cheng, M.-C. et al. (1999) *Angew. Chem. Int. Ed. Engl.,* **38**, 686-689; and Zhang, Y. et al. (1999) *J. Biol. Chem.,* **274**, 6183-6189), the carboxyl group of the non-reducing end sialic acid may exist as an open form.

For the mass-spectral analysis of the enzyme reaction product, the cell-free reaction was carried out using 40 K Sup. The reaction mixture contained 1 mM sialic acid and 10 mM CMP-NeuAc as donor in a reaction buffer containing 25 mM Tris-HCl (pH 8.0), 5 mM MgCl₂, 5 mM DTT, 0.5% Triton CF-54. To drive the forward reaction, the liberated CMP was removed by the addition of alkaline phosphatase (1 U). After the reaction was over, the reaction material was passed through an Amicon filter (10 MWCO) and the filtrate was used for analysis. The cell-free enzyme reaction product behaved similar to the pentameric sialic acid (Calbiochem), on TLC. in various solvent systems. Mass spectra were compared with that of commercially available pentameric sialic acid. Negative ion mass spectrum (ES-MS) of : (A) standard pentameric polysialic acid, and (B) cell-free reaction product. The major peaks for *m*/*z* were marked by arrow and described in the text. The molecular ion (M - H⁺)⁻ was also shown. Some minor products with higher mass (m/z) were also observed.

Mass-spectral analysis of the enzyme reaction products was performed with increasing concentration of CMP-NeuAc. The cell-free enzyme reactions were performed as above with 1 mM sialic acid and increasing concentration of CMP-NeuAc ( 10 mM, 15 mM, 20 mM, and 30 mM). After the reaction was complete, the materials were filtered (Amicon; 10 MWCO) and the filtrate from each reaction tube was directly used for mass-spectrometry (MALDI-MS). The spectra were: A, standard pentamer (Calbiochem), *B*, reaction product using 10 mM CMP-NeuAc and C, reaction product using 20 mM CMP-NeuAc. The major peaks for *m*/*z* were shown by arrow and described in the text. A similar spectrum (as C) was observed using 30 mM CMP-NeuAc.

¹³C-NMR analysis was performed on the capsular polysaccharide. The spectra were recorded on a Brucker DRX-600 spectrometer. The scale is in parts per million and relative to external DMSO. The spectra of the purified capsular polysaccharies were from K1 (A), K1 transformed with K92*neuS (B),* and K92 (C). The numbering of the carbon atoms is based on the previously published results (ibid, Egan, W. 1977).

¹H -NMR analysis was performed on the capsular polysaccharide of K1 transformed with *K92*/*neuS* gene. The spectra were recorded on a Brucker DRX-600 at an ambient temperature. Only the region for amide methyl groups from α2,8- and α2,9-linkages of the purified capsular polysaccharide have been shown for comparison. The isolation and purification of the capsular polysaccharides were mentioned in the *Materials and Methods.* The spectra of the capsular polysaccharides were from K1 (A), K92 (B), and K1 transformed with K92/neuS (C). While the K1 sample showed only a singlet at δ1.99 K92 showed two peaks at δ2.00 and δ1.96. The spectrum obtained from the transformed K1 was broad and complex, especially at ~δ1.99.

¹³C-NMR analysis of the enzyme reaction product was performed. After the enzyme reaction, the protein was removed by filtration (Amicon; 10K). The enzyme reaction product was separated from unreacted sialic acid and CMP-NeuAc by passing through a column of Dowex 1X8 (PO₄⁻ form) eluting with phosphate buffer. The product was collected in the void volume and desalted by passing through a small column of Sephadex G-50 eluting with water. The desired material was collected in the void volume, freeze-dried, and dissolved in D₂O for NMR analysis. The spectra were recorded on a Brucker DRX-600 spectrometer at an ambient temperature (20 °C). The scale is in parts per million and relative to external DMSO. Spectrum A, commercially available (-2,8-linked pentameric sialic acid used as standard, results from approximately 25K accumulations. Spectrum B, partially purified cell-free reaction product, results from approximately 40K accumulations.

### Findings:

While sialic acid is ubiquitous in eukaryotes, particularly in mammals (ibid, Tsuji,S. 1996), it is not detected in prokaryotes other than some pathogenic bacteria. These include some *Escerichia coli* strains, some *Neisseria meningitidis* strains and bacteria of the O serotype including Salmonella (ibid, Barry, G.T. 1962 and 1965). Though the presence of polysialyltransferase activity in *E.coli* was reported earlier by Roseman et al (ibid, Kundig,F.D. 1971), the nature of the gene and its product was not clearly demonstrated until recently. The cloned *neuS* gene from K1 showed that its product has similar size but no sequence homology to any of the mammalian sialyltransferases. Expression of the K1 *neuS* gene indicates that this 47 kd protein synthesizes polysialic acid *in vitro* (ibid, Weisgerber, C. 1991). A similar gene was cloned from the K92 strain of *E.coli,* the capsule of which was shown to contain heteropolysialic acid (-8NeuAcα2,9NeuAcα2-) (ibid, Egan,W.1977). This gene product differs by 70 amino acids when compared with that of the *neuS* gene product of the K1 strain (ibid, Vimr, E.R. 1992). Of the observed amino acid replacements, 36 were conservative. On the basis of such homology, it was postulated that the K92 gene evolved from *neuS* of K1 (ibid, Vimr, E.R. 1992). By complementation assay, the K92 *neuS* gene was shown by this group (ibid, Steenbergen, S.M. 1992) to express polysialyltransferase activity yielding a product that was recognized by men C *antiserum in vivo,* suggesting that this gene might produce sialoside with alternating α2,8/2,9-polysialyl linkages. However, because men C antiserum interacts with both homopolysialic acid (-9-NeuAca2-) from *Neisseria meningitidis* Group C and heteropolysialic acid (-8NeuAcα2,9NeuAcα2-) from the K92 strain of *E.coli* (Barry, G. T. (1965) *Bull. Soc. Chim. Biol.* **47**, 529-533.34), structural identification of the cell-free reaction product(s) was warranted. Previously, the expression of the K92 *neuS* gene was not clearly demonstrated, nor was it shown that this gene product catalyzes the synthesis of heteropolysialic acid (-8NeuAcα2,9NeuAcα2-) *in vitro.*

The polysialyltransferase (polyST) encoded by *neuS* in *E.coli* uses CMP-sialic acid as a donor and catalyzes sequential sialylation at the nonreducing end of appropriate acceptors. Using K1 *neuS,* the polymerase activity was detected *in vitro* with sialyloligomers (designated colominic acid) or with certain gangliosides, such as GD₃ (Weisgerber, C. 1991) as substrates. However, it was shown previously that this gene product could not initiate *de novo* polysialic acid synthesis (ibid, Steenbergen, S.M. 1992), indicating a requirement for an as yet undefined *in vivo* primer. This primer, or polymerization initiator, does not appear to be colominic acid, since this polymer was neither elongated efficiently *in vitro* ( 18) nor the elongated product *detected in vivo* (Barry, G. T., et al., (1962) *J. Gen. Microbiol.* **29**, 335-352). Using K92 *neuS,* we have found that colominic acid is not an acceptor. In fact, among the acceptors tested, only sialic acid and certain gangliosides proved to be good acceptors.

In conclusion, it is disclosed herein that the *neuS* gene product from *E.coli* K92 exhibits α2,8/2,9-polysialyltransferase activity both *in vitro* and *in vivo.* Apparently a single polypeptide (as judged by the Western blot) for this polysialyltransferase catalyzes the synthesis of polysialic acid by sequentially transferring sialic acids with alternating α2,9- and α2,8- linkages. Previously, bacterial KDO-transferase was shown to possess such dual linkage specificity (Glode, M.P., et al., (1977) *J. Infect. Diseases,* **135**, 94-102). Thus, enzymes capable of forming multiple different linkages may not be uncommon in nature. Though the membrane-bound K92 neuS enzyme accepted exogenous neuraminic acid as the best substrate in this study, it may prefer other sialic acid-containing glycolipids or glycoproteins. Work is in progress to define the substrate specificity of this enzyme and to determine the detailed structures of the enzyme products.

### Table I: Substrate specificity of neuS gene product of E.coli K92 expressed in BL21(DE3):

The reaction mixture (60 (l) contained 1 (M each of the following acceptors and 9 nmol of CMP[¹⁴C]NeuAc (specific activity: 8000 cpm/nmol) as donor substrate. The reactions were carried out at 37 °C for 1 hr using 20 (g of 40K Sup as mentioned in 'Materials and Methods.' The reaction mixture (1 (1) was spotted on a plastic backed DEAE-cellulose TLC plate, and ran in *n*-propanol:14.5M NH₄OH:water :: 6:1:3. After air-drying, the plate was exposed to Kodak's XAR-5 film for overnight at -70° C. The area corresponding to the reaction product was marked, cut and then counted in scintillation fluid.

| Potential acceptor | cpm obtained | Total sialic |
|---|---|---|
| substrates tested | (per 60 ul reaction) | acid trans- |
| | | ferred (pmol) |
| Sialic acid (5AcNeuAc) | | |
| Monomer | 3780 | 470 |
| Dimer | 1080 | 140 |
| Trimer | 1560 | 140 |
| Pentamer | 0 | 0 |
| Hexamer | 0 | 0 |
| 9-*O*-acetyl sialic acid | 900 | 110 |
| Colominic acid | 0 | 0 |
| GD₃ | 2880 | 360 |
| GM₃ | 1440 | 180 |
| GD₂ | 900 | 110 |
| GT₁ₐ | 460 | 40 |
| GT_{1b} | 1140 | 130 |
| GQ_{1b} | 2460 | 310 |
| Ganglioside mixtures | 720 | 90 |

## Claims

1. DNA plasmid containing the neuS gene from *escherichia coli* K92 and encoding α2,8/2,9-polysialyltransferase having a hexameric histidine tag fused to its N-terminal end from *escherichia coli* K92.

2. A transformed cell having a gene encoding α2,8/2,9-polysialyltransferase having a hexameric histidine tag fused to its N-terminal end from *escherichia coli* K92, said gene being as specified in claim 1.

3. A process for obtaining α2,8/2,9-polysialyltransferase comprising the following steps:
Step A: expressing a gene encoding α2,8/2,9-polysialyltransferase having a hexameric histidine tag fused to its N-terminal end within a transformed cell having said gene for producing said α2,8/2,9-polysialyltransferase; and then
Step B: releasing α2,8/2,9-polysialyltransferase having a hexameric histidine tag fused to its N-terminal end expressed in Step A from the cell.

4. A process according to claim 3 wherein the α2,8/2,9-polysialyltransferase is from *escherichia coli* K92.

5. Recombinant α2,8/2,9-polysialyltransferase having a hexameric histidine tag fused to its N-terminal end, said α2,8/2,9-polysialyltransferase being unbound to a cell membrane.

6. Recombinant α2,8/2,9-polysialyltransferase having a hexameric histidine tag fused to its N-terminal end according to claim 5 wherein the α2,8/2,9-polysialyltransferase is from *escherichia coli* K92.

7. A method for converting a substrate of α2,8/2,9-polysialyltransferase into a product, said method comprising the step of contacting the substrate with α2,8/2,9-polysialyltransferase having a hexameric histidine tag fused to its N-terminal end that is unbound to a cell membrane under conditions for promoting enzymic catalysis of a conversion of a substrate into the product.

8. A method according to claim 7 wherein the α2,8/2,9-polysialyltransferase is from *escherichia coli* K92.

## Patentansprüche

1. DNA-Plasmid, welches das neuS-Gen aus Escherichia coli K92 enthält und α2,8/2,9-Polysialyl-Transferase codiert, die ein hexameres Histidin-Tag aus Escherichia coli K92 aufweist, das an ihr N-terminales Ende ankondensiert ist.

2. Transformierte Zelle, die ein Gen aufweist, das α2,8/2,9-Polysialyl-Transferase codiert, die einen hexameren Histidin-Tag aus Escherichia coli K92 aufweist, der an ihr N-terminales Ende ankondensiert ist, wobei es sich bel dem Gen um ein solches nach Anspruch 1 handelt.

3. Verfahren zur Herstellung von α2,B/2,9-Polysialyl-Transferase, das die folgenden Stufen umfasst:
Stufe A: Expression eines Gens, das α218/2.9-Polysialyl-Transferase codiert, die ein hexameres Histidin-Tag aufweist, das an ihr N-terminales Ende ankondensiert ist, innerhalb einer transformierten Zelle, die das Gen aufweist zur Herstellung von α(2,8/2,9-Polysialyl-Transferase; und danach
Stufe B: Freisetzung von α2,8/2,9-Polysialyl-Transferase, die ein hexameres Hlstidin-Tag aufweist, das an ihr N-terminales Ende ankondensiert ist, die in Stufe A aus der Zelle exprimiert worden ist.

4. Verfahren nach Anspruch 3, in dem die **α**2,8/2,9-Polysialyl-Transferase aus Escherichia coli K92 stammt.

5. Rekombinante α2,8/2,9-Polysialyl-Transferase, die ein hexameres Histidin-Tag aufweist, das an ihr N-terminales Ende ankondensiert ist, wobei die α2,8/2,9-Polysialyl-Transferase frei gegenüber einer Zellmembran ist.

6. Rekombinante α2,8/2,9-Polysialyl-Transferase, die ein hexameres Histidin-Tag aufweist, das an ihr N-terminales Ende ankondensiert ist, nach Anspruch 6, wobei die α2,8/2,9-Polysialyl-Transferase aus Escherichia coli K92 stammt.

7. Verfahren zur Umwandlung eines Substrats von α2,8/2,9-Polysialyl-Transferase in ein Produkt, wobei das Verfahren umfasst eine Stufe, in der das Substrat mit α2,8/2,9-Polysialyl-Transferase in Kontakt gebracht wird, die ein hexameres Histidin-Tag aufweist, das an ihr N-terminales Ende ankodensiert ist, das gegenüber einer Zellmembran unter solchen Bedingungen frei ist, weiche die enzymatische Katalyse einer Umwandlung eines Substrats in das Produkt fördern.

8. Verfahren nach Anspruch 7, in dem die α2,8/2,9-Polysialyl-Transferase aus Escherichia coli K92 stammt.

## Revendications

1. Plasmide d'ADN contenant le gène neuS d*'Escherichia coli* K92 et codant pour une α2,8/2,9-polysialyltransférase présentant une étiquette histidine hexamère fusionnée à son extrémité N d'*Escherichia* coli K92.

2. Cellule transformée présentant un gène codant pour une α2,8/2,9-polysialyltransférase présentant une étiquette histidine hexamère fusionnée à son extrémité N d*'Escherichia coli* K92, ledit gène étant tel que spécifié à la revendication 1.

3. Procédé d'obtention d'une α2,8/2,9-polysialyltransférase comprenant les étapes suivantes :
Etape A : expression d'un gène codant pour une α2,8/2,9-polysialyltransférase présentant une étiquette histidine hexamère fusionnée à son extrémité N dans une cellule transformée présentant ledit gène pour produire ladite α2,8/2,9-polysialyltransférase; puis
Etape B : libération d'une α2,8/2,9-polysialyltransférase présentant une étiquette histidine hexamère fusionnée à son extrémité N exprimée à l'étape A à partir de la cellule.

4. Procédé suivant la revendication 3, dans lequel 1'α2, 8/2,9-polysialyltransférase provient d*'Escherichia coli* K92.

5. α2,8/2,9-polysialyltransférase recombinée présentant une étiquette histidine hexamère fusionnée à son extrémité N, ladite α2, 8/2, 9-polysialyltransférase étant non liée à une membrane cellulaire.

6. α2,8/2,9-polysialyltransférase recombinée présentant une étiquette histidine hexamère fusionnée à son extrémité N suivant la revendication 5, dans laquelle l'α2,8/2,9-polysialyltransférase provient d*'Escherichia coli* K92.

7. Procédé de conversion d'un substrat d'une α2,8/2,9-polysialyltransférase en un produit, ledit procédé comprenant l'étape de mise en contact du substrat avec une α2,8/2,9-polysialyltransférase présentant une étiquette histidine hexamère fusionnée à son extrémité N qui n'est pas liée à une membrane cellulaire dans des conditions promouvant une catalyse enzymatique d'une conversion d'un substrat en le produit.

8. Procédé suivant la revendication 7, dans lequel l'α2,8/2,9-polysialyltransférase provient d*'Escherichia coli* K92.
